# EUROPÄISCHE PATENTSCHRIFT

(11) **EP 1 347 295 B1**
(45) Veröffentlichungstag und Bekanntmachung des Hinweises auf die Patenterteilung: **13.08.2008**
(21) Anmeldenummer: 02006200.6
(22) Anmeldetag: 19.03.2002
(51) Int. Cl.: G01N 33/42, G01N 33/28

(54) **Verfahren zum Nachweisen oder quantitativen Bestimmen von Naturbitumen in einer Asphaltprobe**
Method for detecting or quantitatively determining Natural bitumen in an asphalt sample
Procédé de détection ou de détermination quantitative de bitume naturel dans un échantillon d'asphalte

(43) Veröffentlichungstag der Anmeldung: 24.09.2003
(73) Patentinhaber: Carl Ungewitter Trinidad Lake Asphalt GmbH & Co. KG, 28195 Bremen (DE)
(72) Erfinder: Lüdersdorf, Reinhard Dr.rer.nat., 12105 Berlin (DE); Kelm, H.-Werner, 10967 Berlin (DE)
(74) Vertreter: Eisenführ, Speiser & Partner

(56) Entgegenhaltungen:
- DE-A- 10 004 402
- DE-A- 19 845 474
- BARAKAT ASSEM O ET AL: "Application of a multimolecular marker approach to fingerprint petroleum pollution in the marine environment." MARINE POLLUTION BULLETIN, Bd. 38, Nr. 7, Juli 1999 (1999-07), Seiten 535-544, XP002212093 ISSN: 0025-326X
- RULLKOETTER J ET AL: "MICROBIAL ALTERATION OF 17-ALPHA H HOPANES IN MADAGASCAR ASPHALTS REMOVAL OF CARBON 10 METHYL GROUP AND RING OPENING" GEOCHIMICA ET COSMOCHIMICA ACTA, Bd. 46, Nr. 9, 1982, Seiten 1545-1553, XP002212094 ISSN: 0016-7037
- SUMMONS R E ET AL: "DISTINCTIVE HYDROCARBON BIOMARKERS FROM FOSSILIFEROUS SEDIMENT OF THE LATE PROTEROZOIC WALCOTT MEMBER CHUAR GROUP GRAND CANYON ARIZONA USA" GEOCHIMICA ET COSMOCHIMICA ACTA, Bd. 52, Nr. 11, 1988, Seiten 2625-2637, XP002212095 ISSN: 0016-7037

## Beschreibung

Die Erfindung betrifft ein Verfahren zum Nachweisen oder quantitativen Bestimmen von Naturasphalt in einer Asphaltprobe.

Straßenasphalt ist ein Gemisch aus Bitumen oder bitumenhaltigen Bindemitteln und Mineralstoffen sowie gegebenenfalls gegebenen Zuschlägen oder Zusätzen. Man unterscheidet zwischen Naturasphalt, bei dem das Bindemittel durch Verdunstung leichtflüchtiger Anteile des Erdöls und oxidative Polymerisation der schwer flüchtigen Rückstände entstanden ist, und industriell gefertigtem Asphalt, bei dem primär petrochemisch hergestelltes Bitumen, synthetisches Erdölbitumen (auch Raffineriebitumen genannt) zum Teil unter Hinzufügen von weiteren Stoffen verwendet wird. Das bekannte vorkommen von Naturasphalt findet sich im Asphaltsee der Insel Trinidad.

Eine wichtige technische Verwendung von Asphalt liegt in der Herstellung von Flächenbefestigungen (Straßen, Flugplätze und dergleichen). Hier gibt es intensive Bemühungen zur Verbesserung der Eigenschaften von Erdölbitumen, beispielsweise in Gestalt polymermodifizierter Bitumen. In der Praxis werden auch regelmäßig Gemische aus synthetischem Bitumen und Naturasphalt eingesetzt, wobei jedoch ein Mindestanteil an Naturasphalt eine wichtige Voraussetzung für ein qualitativ hochwertiges Asphaltgemisch ist. Zur Qualitätsbeurteilung besteht ein Bedarf für ein Verfahren mit dem festgestellt werden kann, ob eine Asphaltprobe Naturasphalt enthält und wie groß dieser Gehalt ist.

Die Druckschrift DE 198 45 474.0 A1 offenbart ein Verfahren zum Nachweisen oder quantitativen Bestimmen eines Zusatzstoffs zu Asphalt, bei dem dem Zusatzstoff eine bekannte Menge eines Tracers beigemischt und im Asphalt der Tracer nachgewiesen oder bei quantitativer Bestimmung sein Gehalt bestimmt und aus dem Tracergehalt des Asphalt dessen Gehalt im Zusatzstoff ermittelt wird. Bevorzugt wird als Tracer eine organische Zinkverbindung eingesetzt, besonders bevorzugt handelt es sich dabei um Zinkdimethyldithiocarmat.
Die Druckschrift DE 100 04 402.6 A1 stellt eine Zusatzanmeldung zur erstgenannten Druckschrift dar, bei der als Tracer Zinkstearat eingesetzt wird.
Barakat Assem 0 et al. offenbaren in dem Artikel "Application of a multimolecular marker approach to fingerprint petroleum pollution in the marine environment", Marine Pollution Bulletin, Bol 38, Nr. 7, Juli 1999 (1999-07), Seiten 535 bis 544, die Anwendung von Biomarkern zur Identifizierung der Quellen petrochemischer Umweltverschmutzungen in Marinen Systemen.
PIERI.N, et. al. offenbaren in dem Artikel "GC-MS identification of biomarks in road asphalts and in their parent crude oils. Relationship between crude oil maturity and asphalt reactivity towards weathering" Org. Geochem, Vol 25, Nr.1/2, 1996, Seiten 51-67 Untersuchungen zur chemischen Analyse von Asphaltproben.
Die Erfindung stellt sich die Aufgabe, ein Verfahren zur Verfügung zu stellen, mit dem ohne Zuhilfenahme eines Tracers der Nachweis und die quantitative Bestimmung von Naturbitumen in einer Asphaltprobe möglich sind, wobei der Naturasphalt im Gemisch mit Erdölbitumen vorliegt.
Diese Aufgabe wird gelöst mit einem Verfahren gemäß Anspruch 1. Weiterbildungen des Verfahrens sind in den Unteransprüchen angegeben.

Erfindungsgemäß wird für den Nachweis und zur quantitativen Bestimmung des Naturasphalts von Biomarkern Gebrauch gemacht, die in Naturasphalt vorkommen, in Erdölbitumen jedoch im wesentlichen nicht vorhanden sind. Die zu analysierende Asphaltprobe wird auf die Anwesenheit oder Menge von solchen Biomarkern untersucht.
Nahfolgend bedeutet der Begriff "Asphaltprobe" eine Probe, so wie sie zu Untersuchungszwecken aus einer der genannten Flächenbefestigungen (beispielsweise aus dem Belag einer Straße oder eines Flugplatzes) entnommen wird. Dieses Material enthält im allgemeinen etwa 10 % Bindemittel und 90 % Zuschlagstoffe (Kies, Sand und dergleichen).

Der auf der Insel Trinidad vorkommende und im Tagebau abgebaute Naturasphalt hat im Mittel einen Bitumenanteil von 54 % und einen Mineralstoffanteil von 46 %. Der Mineratstoffanteil enthält hauptsächlich (mit > 80 %) Partikel der Kornklasse < 0,09 mm. Er kommt, von freiem Wasser und diversen Fremdstoffen befreit, unter dem Namen "Trinidad Epuré" (TE) in den Handel. Nachfolgend wird dieser Name synonym mit dem Begriff "Naturbitumen" verwendet.

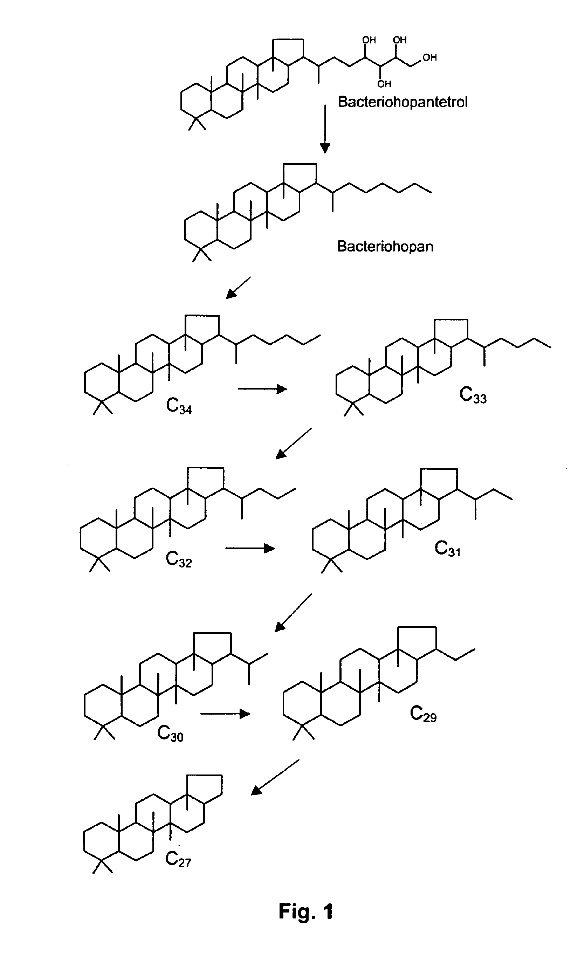

Erdöle wurden über geologische Zeiträume durch mikrobiologische, chemische und thermische Umwandlung von abgestorbenen und sedimentierten Organismen wechselnder Herkunft gebildet, vorwiegend aber aus maritimem Phytoplankton sowie aus der Biomasse der an dessen Abbau beteiligten Bakterien. Das durch Photosynthese (Biosynthese) gebildete Phytoplankton ergab zusammen mit eingetragenen Organismenresten die biogene Komponente der Sedimente. Solche biogene Ausgangsmoleküle lassen sich zurückverfolgen. Die Erdölhopanoide lassen sich auf ein einziges Biohopanoid zurückführen, das Bacteriohopantetrol ( siehe Fig. 1).

Es wurde gefunden, dass sich Geofossilien dieser Art als erfindungsgemäße Biomarker einsetzen lassen. Überraschenderweise enthält Naturasphalt geofossile Kohlenwasserstoffe in bestimmten Verteilungsmustern, die in synthetisch hergestelltem Erdölbitumen unterschiedlicher Provenienz in vergleichbaren Verteilungsmustern nicht auftreten. Obwohl bekannt ist, dass Verteilungsmuster von Geofossilien wie die Hopan- und Steranderivate charakteristisch für Erdöle gemeinsamer geochemischer Herkunft sind, gibt es keinen Versuch, dies im Sinne der vorliegenden Erfindung zu nutzen.

Es ist anzunehmen, dass die Biomarker auf die besondere Entstehungsgeschichte von Naturbitumen zurückzuführen sind. Wird das über einem Erdölvorkommen lastende Deckgebirge soweit erodiert, sickert das Erdöl zur Erdoberfläche. Die leichteren sowie über geologische Zeiträume auch die mittelschweren Anteile des aussickernden Öls verdunsten, und der zutretende Luftsauerstoff führt zu Verharzungs-, Dehydrierungs- und Kondensationsreaktionen. An solchen Ölausbissen verbleiben schwerflüchtige, dunkle und zähe Rückstände. Das Bitumenvorkommen im Asphaltsee auf Trinidad stellt einen solchen Fall dar.

Gemäß einer bevorzugten Ausführungsform der Erfindung wird die Asphaltprobe - wahlweise unter Einsatz von Ultraschall - mit einem Lösemittel extrahiert, welches im wesentlichen selektiv das Bindemittelgemisch löst. Die Untersuchung in Bezug auf die Anwesenheit oder Menge des Biomarkers wird dann in der erhaltenen Extraktionslösung durchführt.

Gemäß einer weiteren bevorzugten Ausführungsform der Erfindung wird die Extraktionslösung gaschromatographisch getrennt und nach der Trennung massenspektrometrisch die Masse eines Massenfragments detektiert, welches für den jeweiligen Biomarker spezifisch ist. Die jeweilige Masse betrifft das Fragment, das bei der massenspektrometrischen Fragmentierung (d.h. bei Beschuss mit 70 eV) mit höchster Intensität auftritt. Dieses Fragment ist nicht unbedingt mit der Masse des Molekülions identisch. Wie in der Massenspektrometrie üblich, wird diese Masse nachfolgend als Verhältnis von Masse zu Ladung, d.h. als Wert "m/z" angegeben.

Geeignete Biomarker sind Hopane, 18 α(H)-Oleane und Gammacerane mit m/z = 191, Methylhopane mit m/z = 205, Sterane mit m/z = 217 und 218, monoaromatische Sterane mit m/z = 253 und triaromatische Sterane mit m/z = 231. Von diesen Massen sind die Fragmente der monoaromatische Sterane mit m/z = 253 derzeit am meisten bevorzugt, gefolgt von den Hopanen, 18 α(H)-Oleanen und Gammaceranen mit m/z = 191.

Die Biomarker stellen Gemische ähnlicher Verbindungen dar, welches gaschromatographisch getrennt wird. Nach der Trennung treten die Komponenten des Gemisches zu unterschiedlichen Retentionszeiten aus der GC-Säule aus, wobei typische Peakmuster auftreten, die für den Biomarker (und damit für Naturbitumen) charakteristisch sind. Jede Komponente des Gemisches (d.h. jeder GC-Peak) kann mit dem jeweiligen Massenfragment (d.h. mit dem jeweiligen Wert von m/z) detektiert werden. Diese Detektion erfolgt massenspektrometrisch, wobei das Massenspektrometer auf die Massen der jeweiligen Fragmente eingestellt wird. Es liefert ein proportionales Signal und zeichnet das Gaschromatogramm auf. Die quantitative Auswertung erfolgt über die Peakflächen.

Ein wichtiger Parameter bei der massenspektrometrischen Detektion stellt die eingesetzte lonisierungsenergie dar. Der üblicherweise in der Praxis dafür eingesetzte Wert beträgt 70 eV. Da die massenspektrometrische Fragmentierung stark von der lonisierungsenergie abhängt, kann eine Veränderung dieses Parameters zu einer Veränderung der Signalausbeute führen. Im Rahmen der vorliegenden Untersuchungen wurde mit 70 eV gearbeitet.

Bevorzugt werden vor die gaschromatographische Trennung weitere chromatographische und andere Reinigungsschritte gesetzt. Dazu gehören die Ausfällung der im Naturasphalt enthaltenen Asphaltene und eine sich daran anschließende säulenchromatographische Behandlung. Einzelheizen hierzu wrden an späterer Stelle beschrieben.

Weitere Merkmale, Vorteile und Anwendungsmöglichkeiten der Erfindung ergeben sich aus der nachfolgenden Beschreibung einer bevorzugten Ausführungsform zusammen mit den Zeichnungen; es zeigen:
- Fig. 1:: die Struktur von Bacteriohopantetrol. sowie in schematischer Darstellung den Übergang zu Bacteriohopan und die Struktur der daraus entstehenden polycyclischen Kohlenwasserstoffe bei schrittweiser Verkürzung der Seitenkette um ein oder zwei Kohlenstoffatome;
- Fig. 2:: die Struktur wichtiger Biomarker von Naturbitumen und die Massenfragmente, mit denen sie massenspektrometrisch detektierbar sind;
- Fig. 3:: das Gaschromatogramm der Biomarker von Naturbitumen bei massenspektrometrischer Detektion durch die Masse m/z = 191 im Bereich der Retentionszeit (Retentionsbereich) von 9,5 bis 18,50 min im Vergleich zum Gaschromatogramm eines Erdölbitumens (Shell B 30/45);
- Fig. 4:: das Gaschromatogramm der Biomarker von Naturbitumen bei massenspektrometrischer Detektion durch die Masse m/z = 253 im Retentionsbereich von 8,86 bis 20,91 min;
- Fig. 5:: einen vergrößerten Ausschnitt aus Fig. 4 im Retentionsbereich von 12,29 bis 15,32 min;
- Fig. 6:: ein Flussdiagramm für die Probenvorbereitung für die erfindungsgemäße quantitative Bestimmung von Naturbitumen; und
- Fig. 7:: beispielhafte, durch lineare Regression erhaltene Kalibriergeraden für das in Fig. 6 dargestellte Verfahren.

Fig. 1 zeigt in schematischer Darstellung den Übergang von Bacteriohopantetrol zu Bacteriohopan sowie die Struktur der daraus entstehenden polycyclischen Kohlenwasserstoffe bei schrittweiser Verkürzung der Seitenkette um ein (bis C₂₉) bzw. zwei Kohlenstoffatome.

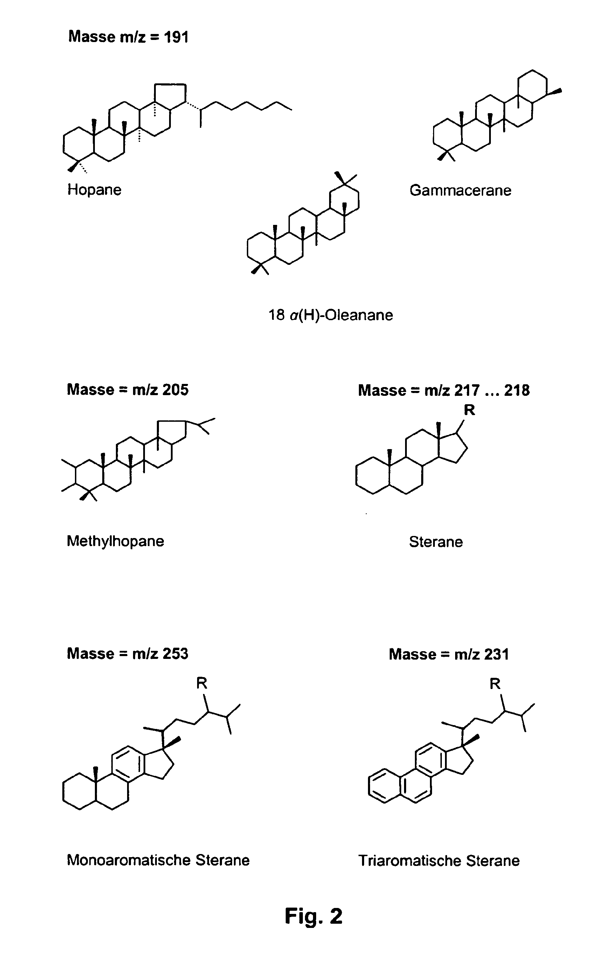

Fig. 2 zeigt die bereits erwähnten Biomarker. Angegeben sind die Strukturen der Hopane, 18 α(H)-Oleane und Gammacerane mit m/z = 191, der Methylhopane mit m/z = 205, der Sterane mit m/z = 217 und 218 sowie der monoaromatischen bzw. triaromatischen Sterane mit m/z = 253 bzw. m/z = 231. Es wurde schon erwähnt, dass von diesen Massen die Fragmente der monoaromatische Sterane mit m/z = 253 derzeit am meisten bevorzugt sind, gefolgt von den Hopanen, 18 α(H)-Oleanen und Gammaceranen mit m/z = 191.

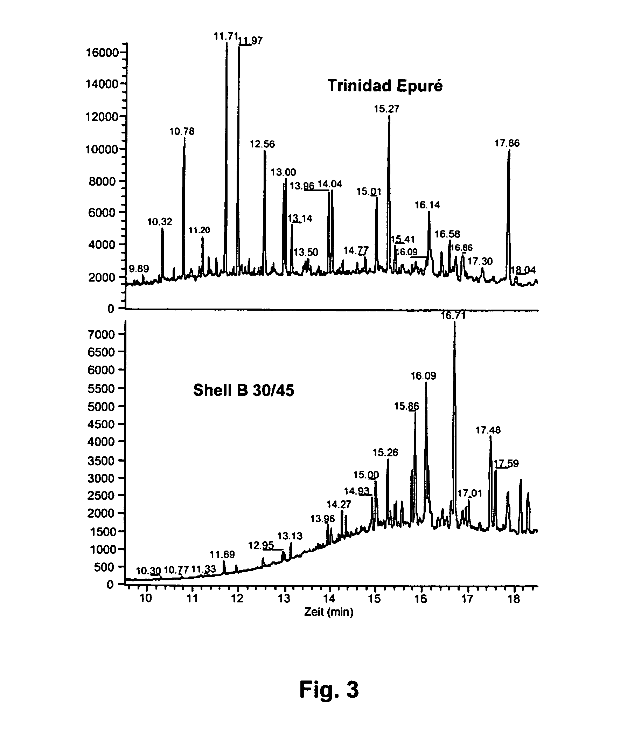

Fig. 3 zeigt das Gaschromatogramm der Biomarker von Naturbitumen bei massenspektrometrischer Detektion durch die Masse m/z = 191 im Bereich der Retentionszeit (Retentionsbereich) von 9,5 bis 18,50 min. Bei diesen und allen anderen Gaschromatogrammen bedeutet die Angabe einer bestimmten Masse, dass die Detektion im Bereich von ± 0,5 Masseneinheiten erfolgt, vorliegend also im Bereich von 190,5 bis 191,5. Zum Vergleich ist auch das Gaschromatogramm eines Erdölbitumens (Shell B 30/45) wiedergegeben. Wie zu erkennen ist, existieren für TE im Retentionsbereich von 9,5 bis 13 min charakterirische Peakmuster, die beim Erdölbitumen nicht auftreten.

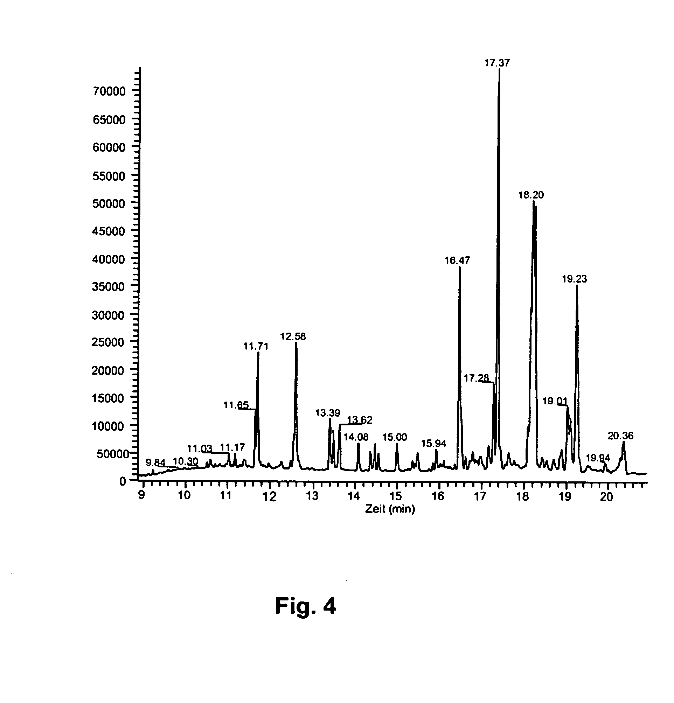

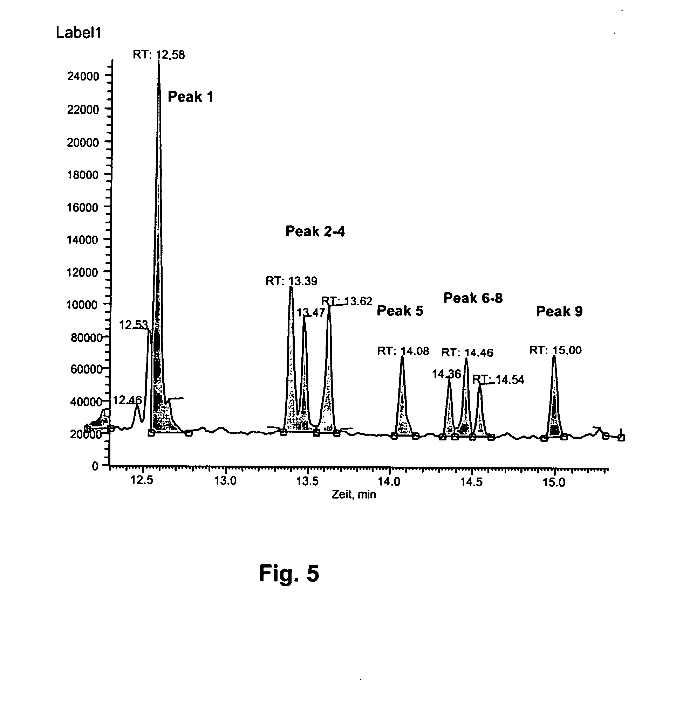

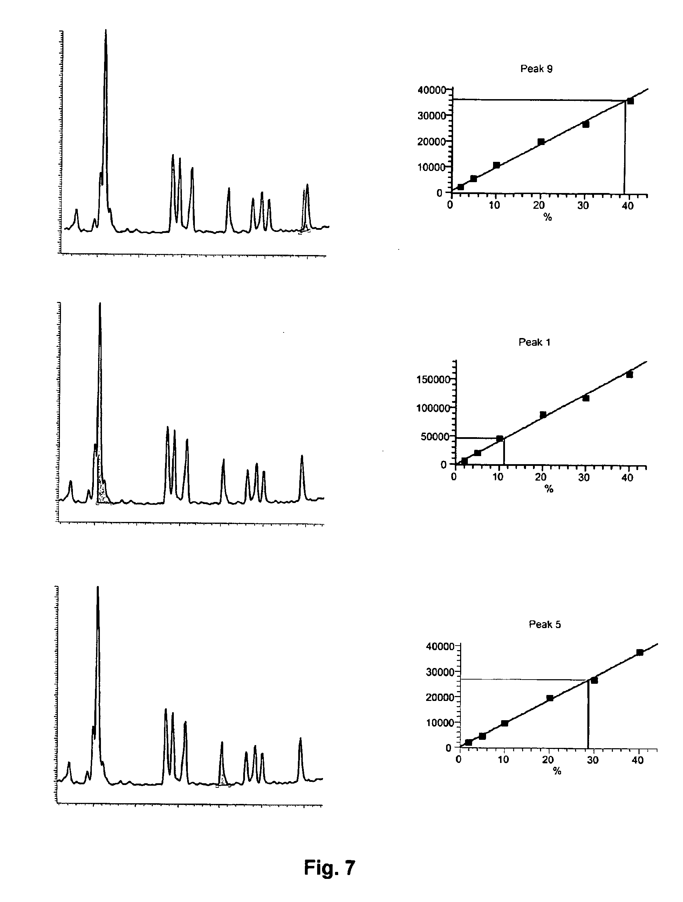

Fig. 4 zeigt das Gaschromatogramm der Biomarker von Naturbitumen bei massenspektrometrischer Detektion durch die Masse m/z = 253 im Retentionsbereich von 8,86 bis 20,91 min. Es findet sich eine charakteristisches Peakmuster im Retentionsbereich von 12,58 bis 15,00 min, welches ebenfalls in Erdölbitumen der unterschiechlichten Provenienzen nicht auftritt. Das Muster ist in Fig. 5 in vergrößerter Darstellung wiedergegeben. Die einzelnen Peaks sind nummeriert, wobei die Peaks Nr. 1, 5 und 9 an späterer Stelle für praktische Messungen verwendet wurden (siehe Fig. 7).

Die Flächen der in Fig. 5 gezeigten Peak stehen zueinander in einem charakteristischen und weitgehend konstantem Verhältnis. Die relativen Peakflächen wurden ermittelt und sind in Tab. 1 wiedergeben.

Durch umfangreiche Vergleichmessungen wurde sichergestellt, dass die Peakmuster von Fig. 5 (m/z = 253) in Edölbitumen unterschiedlicher Provenienzen nicht auftreten. Die überprüften Erdölbitumen sind in Tab. 2 zusammengestellt.

Dieser Befund belegt, dass das erfindungsgemäße Verfahren störungsfreie Ergebnisse liefert.

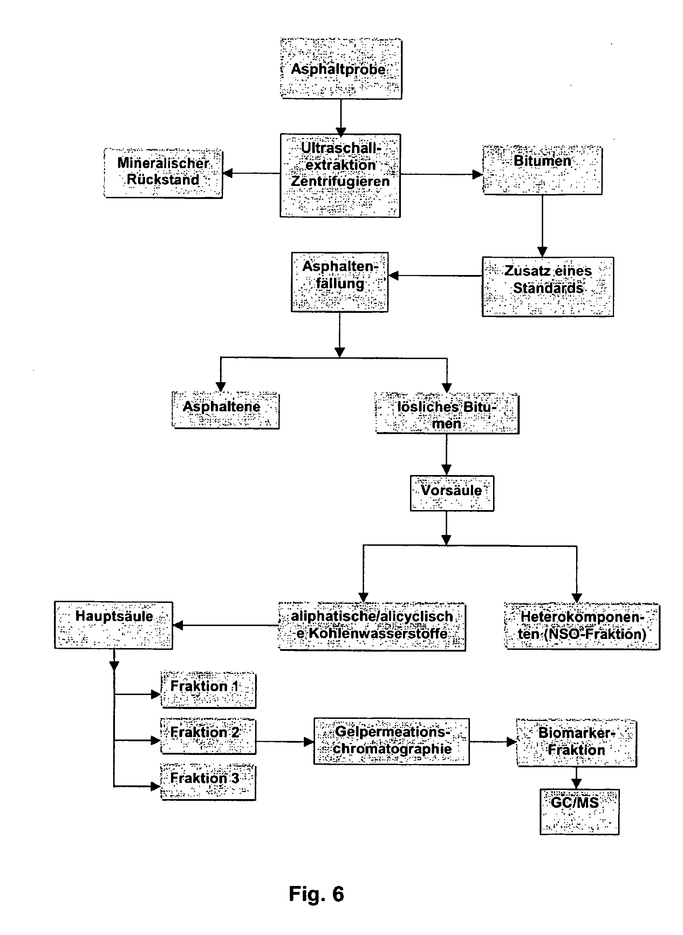

Das nachfolgende Beispiel beschreibt die Durchführung der Bestimmung von Naturbitumen (TE) in einer Asphaltprobe. Das Verfahren ist in Fig. 6 zusammenfassend dargestellt. Soweit Dichlormethan, Hexan oder Methanol zum Einsatz kamen, wurde die Qualität "Picograd" der Fa. Promochem verwendet.

### 1. Extraktion der Probe

Etwa 50 g der Asphaltprobe wurden genau eingewogen und in 250 ml Dichlormethan:Methanol (99:1) 15 min lang im Ultraschallbad behandelt. Die Mischung wurde quantitativ in Zentrifugenflaschen überführt und 10 min bei 3.500 min⁻¹ zentrifugiert. Die überstehende Lösung wurde in einen Rundkolben dekantiert. Der Rückstand wurde nochmals mit 50 ml Dichlormethan aufgeschlämmt, im Ultraschallbad behandelt und wie beschrieben zentrifugiert. Die überstehende Lösung wurde zu der ersten Lösung gegeben und in einem Rotationsverdampfer eingeengt. Der Rückstand wurde mit Dichlormethan in einen Meßkolben mit 25 ml bis zur Meßmarke aufgefüllt.

Der abzentrifugierte Rückstand wurde getrocknet und ausgewogen. Der Bindemittelgehalt (d.h. der in Dichlormethan lösliche Anteil) ergibt sich aus der Differenz zwischen Einwaage der Asphaltprobe und dem unlöslichen Rückstand. Der Bindemittelanteil wird in Gew.-% angegeben.

### 2. Asphaltenfällung

Von der in Dichlormethan gelösten Bitumenlösung wurde eine Menge Lösung abgenommen, die 1 g Bitumen entspricht. Sowie relevant, kann dieser Lösung ein Wiederfindungsstandard in Gestalt von 100 µl einer definierten Squalanlösung zugegeben werden. Dann werden zur Fällung der in Hexan unlöslichen Asphaltene 30 ml Hexan pro ml Bitumenlösung vorgelegt. Nach Eintrag der abgenommenen Bitumenlösung in das Hexan wurde 2 min lang im Ultraschallbad behandelt, die Suspension quantitativ in eine Zentrifugenflasche überführt und 10 min lang bei 3.500 min⁻¹ zentrifugiert. Die überstehende Lösung wurde in einen Rundkolben dekantiert.

Zum Rückstand wurden 20 ml Hexan gegeben, nach kräftigem Schütteln kurz im Ultraschallbad behandelt und nochmals zentrifugiert. Die abdekantierte Waschlösung wurde der ersten Lösung zugeschlagen. Die erste Waschlösung sollte nur leicht verfärbt sein, ansonsten ist der Waschvorgang wie beschrieben zu wiederholen. Der Asphaltenrückstand wurde durch Lösen in Dichlormethan quantitativ in einen Rundkolben überführt und zur Trockne eingeengt.

Die vereinigten Hexanlösungen wurden am Rotationsverdampfer bei 40 °C im kontrollierten Vakuum zur Trockne eingeengt und der gesamte Rückstand für den nachfolgenden Trennungsgang über Kieselgel (Vorsäule) eingesetzt.

### 3. Trennung über die Vorsäule

Eingesetzt wurden Silicagel 100 der Fa. Fluka (200 bis 400 mesh, 0,04 bis 0,063 mm), welches 2 h lang bei 600 °C aktiviert war, Seesand p.A. und Natriumsulfat p.A. der Fa. Merck.

In eine Glassäule (Länge 28 cm, Innendurchmesser 10 mm) mit Fritte wurden auf 1 cm Seesand 9 g aktiviertes Silicagel mittels Hexan eingeschlämmt (Füllhöhe 23 cm). Nach Absetzen des Silicagels wurde es mit bei 500 °C getrocknetem Natriumsulfat überschichtet. Nach Spülen der Säule mit Hexan wurde der hexanlösliche Rückstand aus der Asphaltenfällung auf die Trennsäule überführt und unter leichtem Überdruck mit 80 ml Hexan eluiert. Die Hexanfraktion enthält die aliphatischen, acyclischen und aromatischen Kohlenwasserstoffe.

Eine zweite Fraktion wurde durch Elution mit 60 ml Dichlormethan:Methanol gewonnen. Dieses Eluat enthält die sogenannten NSO-Verbindungen (Stickstoff-, Schwefel- und Sauerstoffhaltige Verbindungen). Die NSO-Fraktion wurde am Rotationsverdampfer im kontrollierten Vakuum zur Trockne eingeengt.

Die Hexanfraktion wurde am Rotationsverdampfer im kontrollierten Vakuum zur weiteren chromatographischen Fraktionierung über eine Kieselgel-Drucksäule (Hauptsäule) aufkonzentriert.

### 4. Trennung über die Hauptsäule

Eingesetzt wurden eine LC-Glas-Drucksäule (Latek, Länge 58 cm, Innendurchmesser 24,4 mm) mit Temperiermantel sowie Probenaufgabeventil, Probenschleife (5 ml, Latek) und Sechswege-Schaltventil. Als Pumpe diente eine HPLC-Gradientenpumpe der Fa. Waters. Als Säulenfüllung diente Kieselgel 60 der Fa. Merck (230 bis 400 mesh, 0,04 bis 0,063 mm), über Nacht bei 150 °C aktiviert.

Eine Glasdrucksäule wurde durch Einschlämmen von Kieselgel 60 in einer Höhe von 400 mm befüllt. Nach Befestigung der Verteilerstempel und einer Dichtigkeitskontrolle wurde die Säule mittels der HPLC-Pumpe mit 300 ml Hexan gespült (10ml/min). Über das Probenaufgabeventil wurde die Probenschleife mit dem Rückstand der Hexanfraktion der Vorsäule gefüllt, auf die Trennsäule aufgegeben und mit Hexan eluiert (10ml/min). Nach einem Durchfluss von 170 ml Hexan wurde Fraktion 1 abgenommen, Fraktion 2 nach Elution von weiteren 250 ml Hexan. Fraktion 1 enthielt verzweigte und unverzweigte aliphatische Kohlenwasserstoffe, Hopane und Oleane. In dieser Fraktion befindet sich auch ggf. das als Wiederfindungsstandard zugesetzte Squalan. Fraktion 2 enthielt unter anderen die für die Identifizierung von Trinidad Epure geeigneten Biomarker. Fraktion 1 und 2 wurden jeweils am Rotationsverdampfer im kontrollierten Vakuum zur Trockne eingeengt, der Rückstand in je einen Messkolben mit 2 ml überführt und zur Messmarke mit Hexan aufgefüllt.

Durch Umleitung des Eluenten über ein Schaltventil auf das andere Säulenende (Backflush) wurden durch Elution mit 250 ml Hexan:Dichlormethan (6:4) auf der Säule verbliebene aromatische Kohlenwasserstoffe eluiert (Fraktion 3). Nach Spülen der Säule mit 250 ml Hexan konnte die nächste Probe chromatographiert werden.

### 5. Trennung über Gelpermeationschromatographie

Für die GC/MS-Messungen zur Erfassung der Trinidad Epuré spezifischen Biomarker musste das als Fraktion 2 genommene Hexaneluat der Hauptsäule einer weiteren Aufreinigung unterzogen werden. Diese Fraktion enthielt noch größere Mengen von höhermolekularen Verbindungen, die bei der anschliessenden gaschromatographischen Trennung zu einer zu starken Belastung des zur Trennung eingesetzten gaschromatographischen Systems und damit zu einem hohen Basislinienrauschen geführt hätten. Zur Entfernung dieser Verbindungen wurde die Gelpermeationschromatographie eingesetzt.

Eingesetzt wurden eine Zweigradienten-HPLC-Pumpe, Modell 2700, und ein Einkanal UV-Detektor, Modell 1706, der Fa. Biorad sowie ein Autosampler Gina 160, ein Fraktionssammler Foxi Junior FC144 und ein Säulentemperierofen der Fa. Dionex. Als Chemikalien dienten Tetrahydrofuran HPLC Grade der Fa. Roth. Als Füllmaterial für die Gelsäule diente Phenogel GPC 5 µm der Fa. Phenomenex mit einer Porenweite von 50 Å (MW Range 100 bis 3 K).

Zur Abtrennung von Verbindungen im Molmassenbereich > 500 wurden zweimal 100 µl von Fraktion 2 (Hauptsäule) über eine Phenogel GPC-Säule mit Tetrahydrofuran chromatographiert (1ml/min). Das Eluat wurde über einen Fraktionssammler gesammelt. Wie durch UV-Detektion festgestellt wurde, befanden sich die interessierenden Biomarker in der Fraktion 9 bis 11 min. Diese Fraktion wurde durch Abblasen mit Stickstoff zur Trockne eingeengt, in 500 µl Hexan:Dichlormethan (1:1) aufgenommen und mittels GC/MS analysiert.

### 6. Untersuchung mit GC/MS

Es wurde ein Gaschromatograph der Fa. Agilent mit Autosampler und einem temperaturprogrammiertem EinspritzlocK KAS 4 der Fa. Gerstel verwendet. Als Säule diente eine Kapillarsäule von Agilent (Länge 30 m, Innendurchmesser 0,25 mm, Film 0,25 µm). Als Massenspektrometer diente ein MSD 7073 von Agilent, welches im MID-Modus (multiple ion modus) betrieben wurde. Alle Messungen erfolgten mit einer lonisierungsenergie von 70 eV.

Es wurde eine Kalibrierlösungsreihe hergestellt. Dafür wurde ein Erdölbitumen mit sechs unterschiedlichen mit TE-Konzentrationen dotiert. Die Analysendaten des eingesetzten Erdölbitumen und von Trinidad Epuré sind Tab. 3, die Daten der Kalibrierlösungen Tab. 4 zu entnehmen. Die Bestimmung des Bindemittelanteils in Trinidad Epuré (Tab. 4) erfolgte aus verfahrensbedingten Gründen und in Abweichung von DIN 1996 (Teil 6) durch Extraktion mit Dichlormethan.

Zunächst wurden 10,0 g Trinidad Epuré wie unter Teil 1 beschrieben mit 250 ml Dichlormethan:Methanol (99:1) extrahiert. Die resultierende Lösung wurde nach Aufkonzentrieren in einem Rotationsverdampfer quantitativ in einen Messkolben mit 50 ml überführt und mit Dichlormethan bis zur Messmarke aufgefüllt. Diese Lösung wurde für die Dotierung des Erdölbitumens verwendet.

In entsprechender Weise wurden 20 g Erdölbitumen durch Ultraschallbehandlung in 150 ml Dichlormethan:Methanol gelöst, in einem Rundkolben mit einem Rotationsverdampfer auf ca. 60 ml aufkonzentriert, quantitativ in einen Messkolben mit 100 ml überführt und mit Dichlormethan bis zur Messmarke aufgefüllt.

Für die Herstellung der Kalibrierlösungen wurde jeweils ein aliquoter Teil der Trinidad Epuré-Dotierlösung in einen Messkolben mit 5 ml pipettiert und mit der Erdölbitumenlösung bis zur Messmarke aufgefüllt. Es erfolgte dann die Asphaltenfällung wie unter Ziffer 2 beschrieben. Die gesamte Lösung von 5 ml wurde in 150 ml Hexan eingetragen und wie dort beschrieben aufgearbeitet. Die weitere Probenvorbereitung erfolgte gemäß Ziffer 3 und 4.

Mit den erhaltenen Kalibrierlösungen wurden Kalibriergeraden aufgestellt, die in Fig. 7 zusammen mit den Gaschromatogrammen gezeigt sind. Die Gaschromatogramme sind praktisch identisch mit denen von Fig. 5. Die Auswertung erfolgte parallel über die Peaks 1, 5 und 9 mit den Retentionszeiten 12,58, 14,08 und 15,00 min. Die Auswertung der Peakflächen ergab die drei gezeigten Kalibrierkurven, deren regressionsanalytische Daten in Tab. 5 angegeben sind. Es ergab sich in allen Fällen ein Korrelationskoeffizient (R²) von > 0,99. Es handelt sich also um leistungsfähiges analytisches Verfahren.

Zur praktischen Erprobung des Verfahrens in praktischen Messungen wurden schließlich Erdölbitumenlösungen hergestellt, die mit Dotierlösung Trinidad Epuré dotiert waren, und auf gleiche Weise gemessen wie die Kalibrierlösungen. Die Dotierung erfolgte wie bei den Kalibrierlösungen. Es kamen jeweils zwei Dotierungen zum Einsatz, die als "Konzentration 1" und "Konzentration 2" bezeichnet sind. Die erhaltenen Messergebnisse (Wiederfindungen) sowie die Art der eingesetzten Erdölbitumen ist Tab 5 zu entnehmen. Es wurden jeweils Doppelbestimmungen unter Zugrundelegung der Flächen von Peak 1, 5 und 9 durchgeführt.

Die eingesetzten Dotierungen betragen 5 % TE bei Konzentration 1 und 20 % TE bei Konzentration 2. Sie sind auf Bitumen berechnet. Wenn ein Bindemittelgehalt in einer Asphaltprobe von 10 % zugrundegelegt wird, betragen sie (berechnet auf die Asphaltprobe) 0,5 % bzw. 2 % TE.

**Tabelle 1**

| Peakflächenverhältnisse | |
|---|---|
| Peak Nr. | Wert, % |
| 1 | 100,00 |
| 2 | 36,4 |
| 3 | 24,5 |
| 4 | 31,1 |
| 5 | 19,5 |
| 6 | 11,6 |
| 7 | 18,5 |
| 8 | 11,3 |
| 9 | 20,2 |

**Tabelle 2**

| Überprüfte Erdölbitumen | | |
|---|---|---|
| Provenienz | Sorte | Raffinerie |
| Venezuela | B50/70 | VEBA |
| Venezuela/Deutschland | B30/45 | ELF |
| Russland | PmB45A | VEBA |
| Saudi Arabien | B20/30 | SHELL |
| Mittel Ost | B30/45 | ESSO |
| Mittel Ost | B50/70 | BP |
| Saudi Arabien | B50/70 | ESSO |

**Tabelle 3**

| Analysendaten der eingesetzten Erdölbitumen und Naturbitumen | |
|---|---|
| Erdölbitumen | |
| Sorte | B50/70 |
| Lieferant/Raffinerie | VEBA Ruhr Oel GmbH Gelsenkirchen |
| Provenienz | Venezuela |
| Hexanunlösliche Asphaltene | 13,3 % |
| Aliphatische (acyclische) und aromatische Anteile | 39,2 % |
| NSO-Anteile | 47,5 % |

| Naturasphalt | |
|---|---|
| Sorte | Trinidad Epurè |
| Lieferant/Raffinerie | Trinidad Lake Asphalt |
| Mineralische Anteile | 44,5 % |
| Bindemittel (Bitumen) | 55.5 % |
| Zusammensetzung des Bindemittels | |
| Hexanunlösliche Asphaltene | 45,5 % |
| Aliphatische (acyclische) und Aromatische Anteile | 21,6 % |
| NSO-Anteile | 32,9 % |

**Tabelle 4**

| Daten der Kalibrierlösungen | |
|---|---|
| Bezeichnung der Lösung | TE-Gehalt (bezogen auf Erdölbitumen), % |
| T100 | 2 |
| T250 | 5 |
| T500 | 10 |
| T1000 | 20 |
| T1500 | 30 |
| T2000 | 40 |

**Tabelle 5**

| Daten der Regressionsanalyse | | | |
|---|---|---|---|
| Peak Nr. | 9 | 1 | 5 |
| Achsenabschnitt | 815,6 | -468,478 | 256,322 |
| | 69 | | |
| Steigung | 908,8 | 4140,81 | 924,934 |
| | 38 | | |
| Korrelationskoeffizient (R²) | 0,994 | 0,9941 | 0,9984 |
| | 2 | | |

**Tabelle 6**

| Wiederfindungen | | |
|---|---|---|
| Dotierung (Soll) | Konzentration 1 (berechnet auf Bitumen) | Konzentration 2 (berechnet auf Bitumen) |
| | 5 % TE | 20 % TE |
| Wiederfindung | | |
| (berechnet auf Bitumen Doppelbestimmungen) | | |
| Bitumen 30/45 | | |
| Venezuela-Deutschland | | |
| VEBA | | |
| Peak 1 | 4,95; 4,9 % TE | 18,9; 19,3 % TE |
| Peak 5 | 5,05; 4,95 % TE | 20,1; 20,5 % TE |
| Peak 9 | 4,6, 4;75 % TE | 19,1; 18,7 % TE |
| Bitumen PmB45A | | |
| Russland VEBA | | |
| Peak 1 | 5,10; 5,25 % TE | 20,8; 21,1 % TE |
| Peak 5 | 4,98; 5,15 % TE | 19,5; 20,7 % TE |
| Peak 9 | 5,16; 5,30 % TE | 5,16; 5,39 % |
| Bitumen B20/30 | | |
| Saudi Arabien SHELL | | |
| Peak 1 | 5,71; 5,38 % TE | 19,3; 20,3 % TE |
| Peak 5 | 4,93; 5,23 % TE | 20,9; 19,7 % TE |
| Peak 9 | 5,46; 5,09 % TE | 18,9; 19,3 % TE |

## Patentansprüche

1. Verfahren zum Nachweisen oder quantitativen Bestimmen von Naturasphalt in einer Asphaltprobe, wobei der Naturasphalt in einem Gemisch mit Erdölbitumen und ggf. weiteren Komponenten vorliegt und das Gemisch als Bindemittel für die Asphaltprobe fungiert, **dadurch gekennzeichnet, dass**
man das Bindemittelgemisch der Asphaltprobe auf die Anwesenheit oder Menge von Biomarkern untersucht, die in Naturasphalt vorkommen, in Erdölbitumen jedoch im wesentlichen nicht enthalten sind, wobei die Asphaltprobe, unter Einsatz von Ultraschall, mit einem Lösemittel extrahiert wird, welches im wesentliche selektiv das Bindemittelgemisch löst, und die Untersuchung in der erhaltenen Extraktionslösung durchführt.

2. Verfahren nach Anspruch 1, **dadurch gekennzeichnet, dass**
man die Extraktionslösung gaschromatographisch trennt und nach der Trennung massenspektrometrisch die Masse eines Massenfragments detektiert, welches für den jeweiligen Biomarker spezifisch ist.

3. Verfahren nach Anspruch 2, **dadurch gekennzeichnet, dass**
man mindestens eine der Massen m/z = 191, 205, 217, 218, 231 oder 253 detektiert.

4. Verfahren nach einem der Ansprüche 1 bis 3, **dadurch gekennzeichnet, dass**
man vor der gaschromatographischen Trennung in der Extraktionslösung enthaltene Asphaltene durch Zugabe eines Fällungsmittels ausfällt.

5. Verfahren nach Anspruch 4, **dadurch gekennzeichnet, dass**
man aus dem nach Fällen der Asphaltene verbleibenden Substanzgemisch durch säulenchromatographische Trennung die aliphatischen, alicyclischen und aromatischen Kohlenwasserstoffe abtrennt.

6. Verfahren nach Anspruch 5, **dadurch gekennzeichnet, dass**
man aus den aliphatischen, alicyclischen und aromatischen Kohlenwasserstoffen durch fraktionierte säulenchromatographische Trennung eine die Biomarker enthaltende Fraktion abtrennt.

7. Verfahren nach Anspruch 6, **dadurch gekennzeichnet, dass**
man aus der erhaltenen Fraktion durch gelpermeationschromatographische Trennung - wahlweise durch Kontrolle mit einem UV-Detektor - die Biomarker abtrennt und diese Fraktion gaschromatographisch/massenspektrometisch untersucht.

8. Verfahren nach einem der vorgehenden Ansprüche, **dadurch gekennzeichnet, dass** man die massenpektrometrische Detektion bei einer lonisierungsenergie von 70 eV durchführt.

## Claims

1. A method of detecting or quantitatively determining natural asphalt in an asphalt sample, wherein the natural asphalt is present in a mixture of petroleum bitumen and possibly further components and the mixture functions as a binding agent for the asphalt sample, **characterised**
**in that**
the binding agent mixture of the asphalt sample is investigated for the presence or amount of biomarkers which occur in natural asphalt but are substantially not contained in petroleum bitumen, wherein the asphalt sample, with the use of ultrasound, is extracted with a solvent which substantially selectively dissolves the binding agent mixture and the investigation is carried out in the extraction solution produced.

2. A method according to claim 1 **characterised in that** the extraction solution is subjected to gas chromatography separation and after the separation operation the mass of a mass fragment which is specific for the respective biomarker is detected by a mass spectrometry operation.

3. A method according to claim 2 **characterised in that** at least one of the masses m/z = 191, 205, 217, 218, 231 or 253 is detected.

4. A method according to one of claims 1 to 3 **characterised in that** prior to the gas chromatography separation operation asphaltenes contained in the extraction solution are precipitated by the addition of a precipitation agent.

5. A method according to claim 4 **characterised in that** the aliphatic, alicyclic and aromatic hydrocarbons are separated from the substance mixture remaining after precipitation of the asphaltenes by column chromatography separation.

6. A method according to claim 5 **characterised in that** a fraction containing the biomarkers is separated from the aliphatic, alicyclic and aromatic hydrocarbons by fractionated column chromatography separation.

7. A method according to claim 6 **characterised in that** the biomarkers are separated from the fraction obtained by gel permeation chromatography separation - optionally by monitoring with a UV detector - and said fraction is investigated by gas chromatography/mass spectrometry means.

8. A method according to one of the preceding claims **characterised in that** the mass spectrometry detection operation is carried out with an ionisation energy of 70 eV.

## Revendications

1. Procédé pour la détection ou la détermination quantitative d'asphalte naturel dans un échantillon d'asphalte, dans lequel l'asphalte naturel est présent dans un mélange avec du bitume de pétrole et le cas échéant avec d'autres composants, le mélange agissant en tant que liant pour l'échantillon d'asphalte, **caractérisé en ce que** l'on examine, dans le mélange liant de l'échantillon d'asphalte, la présence de biomarqueurs ou la teneur en biomarqueurs qui existent dans de l'asphalte naturel mais lesquels ne sont essentiellement pas contenu dans du bitume de pétrole, l'échantillon d'asphalte étant extrait, sous utilisation d'ultrason, avec un solvant qui dissout de manière essentiellement sélective le mélange liant, et que l'on réalise l'examen dans la solution d'extraction obtenue.

2. Procédé selon la revendication 1, **caractérisé en ce que** l'on sépare la solution d'extraction par chromatographie en phase gazeuse, et que l'on détecte la masse d'un fragment de masse qui est spécifique à un biomarqueur respectif, par spectrométrie de masse après la séparation.

3. Procédé selon la revendication 2, **caractérisé en ce que** l'on détecte au moins une des masses suivantes m/z =191, 205, 217, 218, 231, ou 253.

4. Procédé selon l'une quelconque des revendications 1 à 3, **caractérisé en ce que** l'on précipite des asphaltes contenus dans la solution d'extraction au moyen d'un ajout d'un agent de précipitation, avant la séparation par chromatographie en phase gazeuse.

5. Procédé selon la revendication 4, **caractérisé en ce que** l'on sépare les hydrocarbures aromatiques, alicycliques et aliphatiques du mélange de substances restant après précipitation des asphaltes, au moyen d'une séparation par chromatographie en colonne.

6. Procédé selon la revendication 5, **caractérisé en ce que** l'on sépare une fraction contenant les biomarqueurs des hydrocarbures aromatiques, alicycliques et aliphatiques, au moyen d'une séparation par chromatographie en colonne fractionnée.

7. Procédé selon la revendication 6, **caractérisé en ce que** l'on sépare les biomarqueurs de la fraction obtenue au moyen d'une séparation par chromatographie d'exclusion stérique - optionnellement au moyen d'un contrôle avec un détecteur-UV - et que l'on examine cette fraction par chromatographie en phase gazeuse/spectrométrie de masse.

8. Procédé selon l'une quelconque des revendications précédentes, **caractérisé en ce que** l'on réalise la détection par spectrométrie de masse avec une énergie d'ionisation de 70 eV.
